(19) Europäisches Patentamt
European Patent Office
Office européen des brevets

(11) EP 1 685 942 A1

(12) EUROPÄISCHE PATENTANMELDUNG

(43) Veröffentlichungstag:
**02.08.2006 Patentblatt 2006/31**

(21) Anmeldenummer: **06100057.6**

(22) Anmeldetag: **04.01.2006**

(51) Int Cl.:
***B29C 47/10*** *(2006.01)* ***B29C 47/40*** *(2006.01)*
***B32B 37/15*** *(2006.01)* ***C09J 7/02*** *(2006.01)*
***A61K 9/70*** *(2006.01)*

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(30) Priorität: **31.01.2005 DE 102005005173**

(71) Anmelder: **Beiersdorf AG**
**20253 Hamburg (DE)**

(72) Erfinder:
- **Bloens, Tobias**
**25482 Appen (DE)**
- **Jauchen, Peter**
**22455 Hamburg (DE)**
- **Leutz, Reiner**
**21465 Reinbek (DE)**
- **Nierle, Jens**
**21147 Hamburg (DE)**

## (54) Wirkstoffhaltige Selbstklebemassen und deren Herstellung

(57) Verfahren zur kontinuierlichen, lösungsmittelfreien Herstellung von Selbstklebemassen aus natürlichen Kautschuken und/oder synthetischen Kautschuken mit mindestens einem pharmazeutischen Wirkstoff durch Compoundierung und deren inline-Beschichtung zu Wirkstoffpflastern und ein entsprechendes Verfahrensprodukt.

Abbildung 1

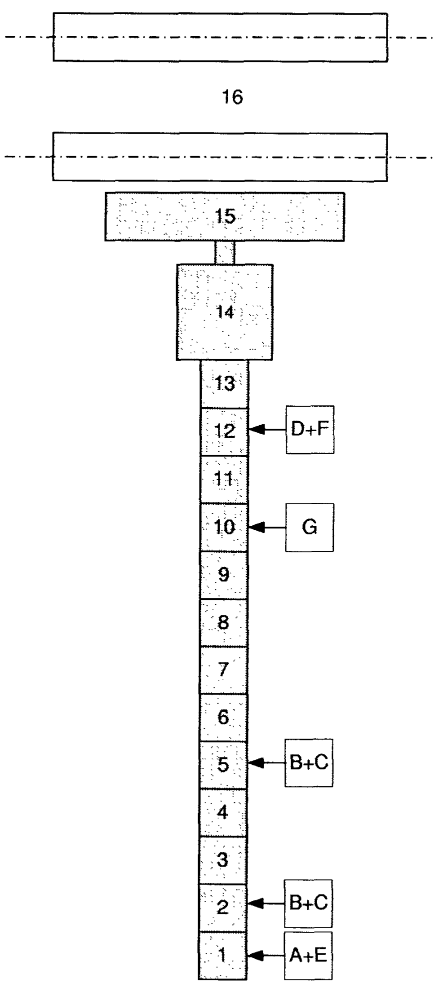


EP 1 685 942 A1

**Beschreibung**

Verfahren zur kontinuierlichen, lösungsmittelfreien Herstellung von Selbstklebemassen aus natürlichen Kautschuken und/oder synthetischen Kautschuken mit mindestens einem pharmazeutischen Wirkstoff durch Compoundierung und deren inline-Beschichtung zu Wirkstoffpflastern und ein entsprechendes Verfahrensprodukt.

Selbstklebende medizinische Trägermaterialien sind seit über 100 Jahren bekannt. Die ersten Wundabdekkungen bestanden aus textilen Trägermaterialien, die mit klebrigen Harzen bestrichen wurde. Im laufe der Zeit entwikkelten sich die medizinischen Trägermaterialien weiter, wobei auch lösungsmittelhaltige Klebstoffe zur Beschichtung verwand wurden. Aus Umwelt- und Arbeitsschutzaspekten ist es jedoch vorteilhaft, lösungsmittelfreie Klebstoffsysteme bei der Herstellung von selbstklebenden Materialien im allgemeinen zu verwenden. Wird auf eine Verwendung von Lösungsmitteln verzichtet, werden keine kostenintensiven Maßnahmen wie Explosionsschutz, Lösungsmittelrückgewinnung und Abluftreinigung benötigt.

Entwickelt wurden daher die so genannten Hotmeltklebemassen, die lösungsmittelfreie Selbstklebemassen darstellen. Die Auftragung erfolgt in der Regel direkt aus einem Extruder, in dem die Einzelkomponenten gemischt und erwärmt werden (Compoundierprozess). Nach dem Erkalten der aufgetragenen Schmelze (Hotmelt) bildet sich eine Selbstklebeschicht aus, deren Klebeeigenschaften von den enthaltenen Komponenten und vom Herstellungsprozess (Compoundierprozess) abhängen.

Für selbstklebende Pflastermaterialien wird gerne auf Hotmeltklebemassen auf Naturkautschukbasis zurückgegriffen. Naturkautschuk ist kostengünstiger als synthetische Polymere und erlaubt über den Abbaugrad während des Herstellungsprozesses eine gezielte Einstellung der Klebeigenschaften, durch die auf der Haut sehr gute Klebeigenschaften erzielt werden.

Naturkautschuk (Kurzbezeichnung nach DIN ISO 1629: 1981-10 ist NR, abgeleitet von *E natur*al rubber) kommt im weißen Milchsaft (Latex) der Milchröhren zahlreicher Dikotyledonen vor. Naturkautschuk wird aber fast ausschließlich (zu nahezu 99 %) aus dem Latex gewonnen, der beim Anritzen der Sekundärrinde der Stämme von Kautschuk- od. Parakautschukbäumen (*Hevea brasiliensis,* Familie Wolfmilchsgewächse, Euphorbiaceae) ausfließt. *Hevea brasiliensis* ist ein ursprünglich im Amazonasgebiet einheimischer Baum der heute in fast allen Tropengebieten Afrikas, Asiens u. Südamerikas in großem Umfang plantagenmäßig angebaut wird. Von anderen kautschukführenden Pflanzen u. Organismen - man fand Naturkautschuk sogar in Pilzen (Reizkern) - werden lediglich nur noch der Guttaperchabaum, der Guayule-Strauch (*Parthenium argentatum),* Kok-Saghys u. *Mimusops balata* (syn. *Manilkara bidentata)* zur Gewinnung von Naturkautschuk in geringem Umfang genutzt. Milchsäfte von verschiedenen einheimischen Gewächsen, z. B. Gänsedistel *(Sonchus oleraceus)* od. Salat *(Lactuca sativa)* enthalten ebenfalls Naturkautschuk, prozentual sogar mehr als die genannten tropischen Gewächse. Bei diesen kleinen krautartigen Pflanzen sind aber die Voraussetzungen für eine ökonomische Naturkautschuk-Gewinnung noch nicht gegeben.

Der Latex wird an Ort u. Stelle mit verschiedenen Mitteln (z B. Essigsäure, Ameisensäure oder durch Elektrophorese) zur Gerinnung gebracht, wobei sich der Roh-Naturkautschuk als feste Masse vom Wasser trennt. Die geronnene Masse (Koagulat) wird zwischen zwei Walzen, die unterschiedliche Drehgeschwindigkeiten haben, unter Wasserzusatz gereinigt, zerrissen, gewaschen, geknetet und zu langen, etwa 1 mm dicken "Fellen" von weißem Crepe-Kautschuk aufgerollt. Um die letzten 10 - 20 % Wasser zu entfernen, hängt man diese Felle in etwa 50 °C warmen Räumen auf. "Smoked Sheet" ist eine andere wichtige Rohkautschuk-Handelssorte. Hier wird der Latex nicht wie bei Crepe-Kautschuk durch Natriumhydrogensulfd-Zusatz konserviert, sondem man stellt vom geronnenen Naturkautschuk etwas dickere Felle her und trocknet und räuchert diese in etwa 50 °C warmen Räucherkammern, in denen man Holz oder Kokosnussschalen abbrennt. Heute überführt man den Latex zur Transportkostenersparnis meist in ein feines Pulver, das nachher vulkanisiert wird. Für Spezialzwecke kommen auch die (allerdings aufkonzentrierten) Latices zum Versand. Naturkautschuk in Form von farblosen oder dunklen Kuchen oder Fellen (Sheets) ist leichter als Wasser, fast geruchsfrei, elastisch und sehr dehnbar: Er kann auf das Fünffache seiner Länge auseinander gezogen werden. Naturkautschuk ist ein ungesättigtes Polymer mit

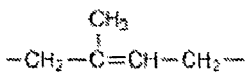

als Grundeinheiten, die in der 1,4-cis-(a; Hevea-Naturkautschuk) bzw. 1,4-*trans*-Konfiguration (b; Balata u. Guttapercha) vorliegen können:

a) 1,4-*cis* - 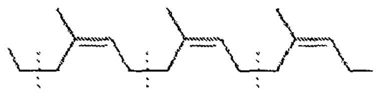 Kautschuk

b) 1,4-*trans* - 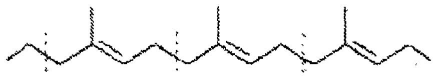 Guttapercha Balata

Naturkautschuk ist also ein Polyisopren, dessen enzymatisch katalysierte Biosynthese über Isopentyl- und Farnesylpyrophosphat als Vorstufen verläuft. Naturkautschuk, Balata und Guttapercha unterscheiden sich zusätzlich durch ihren Polymerisationsgrad, der bei Naturkautschuk ca. 8000-30 000, bei den beiden anderen Kautschuken ca. 1500 beträgt.

Durch die hochmolekularen Anteile im Naturkautschuk (mit $M_w \geq 3*10^5$ g/mol) sind lösungsmittelfreie Naturkautschuk-Selbstklebemassen mit der Schmelzhaftklebertechnologie unverarbeitbar, oder aber die verwendeten Kautschuke müssen vor der Verarbeitung stark in ihrem Molekulargewicht reduziert (abgebaut) werden.

Die übermäßige Mastikation des Polymers muss dabei verhindert werden um eine ausreichende Scherfestigkeit der Selbstklebemasse zu gewährleisten. Anderseits ist ein gewisser Energieeintrag notwendig um die Verarbeitung des Kautschuks zu gewährleisten. Bei der Verarbeitung des Kautschuks zu einer Klebmasse muss sichergestellt sein, dass keine Kautschukpartikel (Stippen) verbleiben.

Die Mastikation (engl.: mastication) stellt eine gummitechnologische Bezeichnung für den Abbau langkettiger Kautschuk-Moleküle zur Erhöhung der Plastizität beziehungsweise Reduzierung der (Mooney-)Viskosität von Kautschuken dar. Die Mastikation wird durchgeführt durch Behandlung insbesondere von Naturkautschuk in Knetern oder zwischen Walzen bei möglichst niedrigen Temperaturen in Gegenwart von Mastikationshilfsmitteln (Mastizierhüfsmittel). Die dabei einwirkenden hohen mechanischen Kräfte führen zu einem "Zerreißen" der Kautschuk-Moleküle unter Bildung von Makroradikalen, deren Rekombination durch Reaktion mit Sauerstoff verhindert wird. Mastikationshilfsmittel wie aromatische oder heterocyclische Mercaptane beziehungsweise deren Zink-Salze oder Disulfide beschleunigen durch Begünstigung der Bildung von Primämadikaten den Mastikationsprozess. Aktivatoren wie Metall- (Eisen-, Kupfer-, Cobalt-) Salze von Tetraazaporphyrinen oder Phthalocyaninen ermöglichen eine Erniedrigung der Mastikationstemperatur. Mastikationshilfsmittel werden bei der Mastikation von Naturkautschuk in Mengen von ca. 0,1 bis 0,5 Gew.-% in Form von Masterbatches eingesetzt, die eine gleichmäßige Verteilung dieser geringen Chemikalienmenge in der Kautschukmasse erleichtern.

Der Mastikationsschritt ist in der Kautschuktechnologie nötig, um eine Erleichterung der Aufnahme der Zusatzstoffe zu erzielen.

Die Klebkraft wird insbesondere bei den Haftklebern auf Basis von Kautschuk durch Einmischen von klebrigmachenden Harzen (Tackifier) und Weichmachern mit relativ niedrigen Molekulargewichten erzielt.

Für das anwendungstechnische Anforderungsprofil von druckempfindlichen Klebesystemen und den damit hergestellten Haftklebeartikeln (wie zum Beispiel Pflaster) sind die zwei physikalischen Phänomene Adhäsion und Kohäsion der Haftkleberschichten von grundsätzlichem Inhalt. Die Adhäsion wird im technischen Sprachgebrauch unter den Begriffen Sofortklebkraft (Tack) und Klebkraft (Peelstrength) behandelt und beschreibt definitionsgemäß die Begriffe "selbstklebend", "Haftkleber" und/oder "druckempfindliche Klebebänder", d. h. die dauerhafte Verklebung unter "leichtem Andruck" ("Pressure Sensitive Adhesives").

Nicht scharf definiert ist die Bezeichnung für Vorgänge des Weichmachens von Kunststoffen unter Anwendung von Scherung (Kneten, Extrudieren, Mastikation), von Temperatur und/oder von Weichmachern (Plasti(fi)ziermittel, Plasti(fi)katoren), wie sie in einem Extruder erfolgen.

Im Sinne dieser Anmeldung ist der Begriff Mastikation streng zu unterscheiden von dem sich bei allen üblichen lösungsmittelfreien Polymertechnologien wie Compoundieren, Fördern und Beschichten in der Schmelze ergebenden Abbau, der Degradation (engl.: degradation).

Die Degradation ist eine Kollektivbezeichnung für unterschiedliche Prozesse, die das Aussehen und die Eigenschaften von Kunststoffen verändern. Degradation kann zum Beispiel durch chemische, thermische, oxidative, mechanische oder biologische Einflüsse oder auch durch Strahleneinwirkung (wie (UV-)Licht) verursacht werden. Folge sind zum Beispiel Oxidation, Kettenspaltungen, Depolymerisation, Vemetzung beziehungsweise Abspaltung von Seitengruppen der Polymeren. Die Stabilität von Polymeren gegenüber einer Degradation kann durch Additive, zum Beispiel durch Zusatz von Stabilisatoren wie Antioxidantien oder Photostabilisatoren erhöht werden.

Die Leistungsfähigkeit des Haftklebers ist also im wesentlichen bestimmt durch das ausgewogene Verhältnis von Adhäsions- und Kohäsions-Eigenschaften und durch Verträglichkeit, Homogenität und Stabilität der Abmischung von Massebestandteilen mit extrem hohen und relativ niedrigen mittleren Molekulargewichten, was bei der Masseherstellung in branchenüblichen Misch- und Knetmaschinen unter Verwendung von Lösemitteln relativ einfach zu erreichen ist.

Wirkstoffhaltige medizinische Trägermaterialien sind wirtschaftlich sehr bedeutsam. Im allgemeinen werden die Wirkstoffe als separate Schichten bzw. Bereiche in die Trägermaterialien eingearbeitet, so das sie nach der Applikation auf der Haut einen direkten Kontakt mit dieser haben (transdermale Pflaster). Um entsprechende Retardwirkungen, das bedeutet Wirkstoffabgabe über längere Zeiträume hinweg, zu erzielen, werden die Wirkstoffe vielfach in spezielle Polymere eingebettet bzw. verkapselt. Die kontinuierliche Herstellung wirkstoffhaltiger (dotierter) medizinische Pflaster aus lösemittelfreier Produktion mit Naturkautschuk als Gerüstpolymer, bei denen die Klebeschicht gleichzeitig die Wirkstoffträgerschicht darstellt, ist bisher nicht beschrieben.

Die internationale Patentanmeldung WO 94/11175 beschreibt ein lösungsmittelfreies kontinuierliches Herstellverfahren. Der Prozess beinhaltet ein Compoundierverfahren mit wechselnden Mastizier- (Knet-) und Mischzonen. Es umfasst jedoch kein dotiertes Hotmeltklebemassesystem.

In der deutschen Patentanmeldung DE 101 37 405 wird ein Verfahren zur kontinuierlichen lösungsmittelfreien und mastikationsfreien Herstellung von Selbstklebemassen auf Basis von SBC mit mindestens einem pharmazeutischen Wirkstoff in einem kontinuierlich arbeitenden Aggregat mit einem Füll- und einem Compoundierteil beschrieben. Die mastikationsfreie Herstellung bedingt den Einsatz von teuren Synthesekautschukmaterialien (SBC) die im Gegensatz zu natürlichem Kautschuk nur eine geringe Quervernetzung aufweisen und daher ohne Mastikation zur Hotmeltmassen verarbeitet werden können.

Die deutsche Patentanmeldung DE 100 01 546 offenbart ein ähnliches Herstellverfahren für polyisobutylenhaltige Hotmelklebemassen.

In der deutschen Patentanmeldung DE 199 39 073 A1 wurde ein anderes Verfahren zur Herstellung von kautschukhaltigen Klebemassen eingesetzt, jedoch ohne eine eigenschaftsschädigende Mastikation im Extruder aufzuweisen. Die Einarbeitung von Wirkstoffen erfolgt nicht.

Das amerikanische Patent US 6,448,303 beschreibt ein Herstellungsverfahren für wirkstoffhaltige Hotmeltklebemassen, jedoch handelt es sich hierbei ebenfalls nicht um einen kontinuierlichen Produktionsprozess.

Zur Compoundierung von Hotmeltklebemassen werden im Normalfall Extruder, insbesondere Zweischneckenextruder verwendet. Als Extruder werden nach DIN 24450: 1987-02 Maschinen bezeichnet, die eine feste bis flüssige Formmassen aufnimmt und aus einer Öffnung vorwiegend kontinuierlich presst (Extrudat). Dabei kann die Formmasse verdichtet, gemischt, plastifiziert, homogenisiert, chemisch umgewandelt, entgast oder begast werden.

Mit diesen beheizbaren, nach dem Prinzip eines Fleischwolfs arbeitenden Maschinen wird die Homogenisierung der eingesetzten Rohstoffe in der Schmelze durchgeführt. Längs der Welle werden folgende Phasen unterschieden:

- Einzugzone, in der die pulverförmigen bzw. granulierte Stoffe über den Extruderzugang in das Innere gezogen und erwärmt werden.

- Plastifizierungszone, in der das Extrudat durch das abnehmende Gangvolumen (verringerter Gehäusedurchmesser) verdichtet, geschmolzen und entlüftet wird.

- Knet- und Homogenisierungszone, in der das Extrudat endgültig homogenisiert und dispergiert wird.

- Durch die formgebende Düse im Spritzkopf wird das Extrudat herausgepresst.

Im wesentlichen werden Einschnecken-Kokneter mit nur einer hubartig fördernden Knetschnecke, Doppelschnecken-Extruder mit einem gegenläufigen oder gleichläufigen Schneckenpaar und Planetwalzen-Extruder mit mehreren um eine große Zentralspindel umlaufenden kleineren Spindein verwendet.

Der Einsatz von ausschließlich nicht-thermoplastischen Kautschuken als Elastomerkomponente in der Haftkleberrezeptierung mit dem bestehenden Kostenvorteil, den zum Beispiel Naturkautschuke gegenüber den handelsüblichen Blockcopolymeren aufweisen, und den herausragenden Eigenschaften, insbesondere der Scherfestigkeit des Naturkautschuks und entsprechender Synthesekautschuke, wird auch in den Patenten WO 94 11 175 A1, WO 95 25 774 A1, WO 97 07 963 A1 und entsprechend US 5,539,033 A, US 5,550,175 A ausführlich dargestellt.

Hierbei werden die in der Haftklebertechnik gebräuchlichen Zusätze wie Tackifier-Harze, Weichmacher und Füllstoffe beschrieben.

Das jeweils offenbarte Herstellungsverfahren basiert auf einem Doppelschneckenextruder, der bei der gewählten Prozessführung über Mastikation des Kautschuks und anschließender stufenweiser Zugabe der einzelnen Zusätze mit einer entsprechenden Temperaturführung die Compoundierung zu einer homogenen Haftkleberabmischung ermöglicht.

Ausführlich wird der dem eigentlichen Herstellprozess vorgeschaltete Mastikationsschritt des Kautschuks beschrieben. Er ist notwendig und charakteristisch für das gewählte Verfahren, da er bei der dort gewählten Technologie

unumgänglich für die nachfolgende Aufnahme der weiteren Komponenten und für die Extrudierbarkeit der fertig abgemischten Masse ist. Beschrieben wird auch die Einspeisung von Luftsauerstoff, wie sie von R. Brzoskowski, J.L. und B. Kalvani in Kunststoffe 80 (8), (1990), S. 922 ff., empfohlen wird, um die Kautschukmastikation zu beschleunigen.

Diese Verfahrensweise macht den nachfolgenden Schritt der Härtung durch Elektronenstrahlvernetzung (ESH) unumgänglich, ebenso wie den Einsatz von reaktiven Substanzen als ESH-Promotoren zum Erzielen einer effektiven Vernetzungsausbeute.

Beide Verfahrensschritte sind in den genannten Dokumenten beschrieben, die gewählten ESH-Promotoren neigen aber auch zu unerwünschten chemischen Vernetzungsreaktionen unter erhöhten Temperaturen. Dies limitiert die Verwendung bestimmter klebrigmachender Harze und den Einsatz der erzeugten Selbstklebemassen insbesondere für pharmazeutische Anwendungen.

Die Verarbeitung von Naturkautschuken zu wirkstoffhaltigen, medizinischen Klebemassen ist technisch sehr schwierig und ist nach dem erfindungsgemäßen Verfahren erstmals möglich.

Die derzeit aus dem Stand der Technik bekannten kontinuierlichen Herstellungsverfahren wirkstoffhaltiger Klebmassen weisen jedoch einige Mängel auf, da sie keinen Naturkautschuk als Gerüstpolymer aufweisen.

Der Verbraucher verbindet mit Naturkautschuken sehr gute Klebeigenschaften auf der Haut. Dies sind eine hohe Anfangsklebrigkeit (Tack), die Repositionierbarkeit des Pflasters sowie die Verklebung auf feuchter Haut. Diese Eigenschaften können mit synthetischen Kautschuken nicht erhalten werden, insbesondere die mangelnde Aufklebkraft und ein eingeschränkte Repositionierbarkeit stellen sich bei der Vermarktung als problematisch dar.

Die nach dem Stand der Technik angewandten zweistufigen Herstellungsverfahren kautschukhaltiger Hotmeltklebmassen unter Verwendung von Lösungsmitteln, wobei erst eine Masseherstellung im Kneter und anschließend eine Beschichtung auf einer Beschichtungsanlage erfolgt, erfordern lange Mischzeiten im Kneter und nach der Beschichtung lange Trocknungszeiten, da die erhaltenen Klebmassen das Lösungsmittel sehr fest einschließen. Während des Knetprozesses wir der Naturkautschuk einer Mastikation unterzogen, das bedeutet zu geringeren Molmassen abgebaut, und mit weiteren Komponenten wie Weichmachern, Klebharzen u.ä. gemischt und im Lösungsmittel gelöst. Ist die Mastikation nicht ausreichend, enthalten die Klebmassen nicht aufgeschlossenen/eingearbeiteten Kautschuk (Stippen), die sich als feste Partikel bemerkbar machen.

Bisher sind auch keine Herstellungsverfahren für naturkautschukhaltige Hotmeltklebemassesysteme bekannt, in die lösungsmittelfrei und kontinuierlich Wirkstoffe eingearbeitet werden. Die bekannten Verfahren erfordern eine vorgeschaltete Mastikation um die Verarbeitung des Naturkautschuks zur ermöglichen.

Bei einer Mastikation ist nach dem derzeitigen Stand der Technik eine anschließende ESH-Vemetzung nötig.

Andere kontinuierliche Verfahren arbeiten bis auf die Füllzone mit einem geschlossenem Aggregat, das bedeutet einem Extruder ohne Seiteneinlässe. Dies lässt eine gezielte Zugabe von festen Rohstoffen (z. B. pulverförmige Füllstoffe und Wirkstoffe) zur schonenden Einarbeitung nicht zu.

Die eingesetzten Wirkstoffsysteme sind in der Regel empfindlich gegen Sauerstoff, Temperatur- und mechanischen Belastung. Dadurch können keine Herstellungsverfahren mit starker Wärmeentwicklung oder Scherung eingesetzt werden. Die üblichen Herstellungsverfahren wie Kneten, Walzen arbeiten mit hohen Temperaturen und Scherbelastungen. Der zum Abfangen der bei der Mastifikation gebildeten Radikale zugeführte Sauerstoff, wirkt sich negativ auf die Wirkstoffe aus und führt zu Klebmassen die nur geringe Mengen an aktivem Wirkstoff enthalten.

Aufgabe der vorliegenden Erfindung ist es, ein Verfahren zur Verfügung zu stellen, mit dem sich Selbstklebemassen auf der Basis von Naturkautschuk, die zumindest einen pharmazeutischen Wirkstoff enthalten, lösemittelfrei kontinuierlich herstellen und gegebenenfalls inline beschichten lassen, ohne dass der Naturkautschuk zuvor mastiziert wird oder eine nachfolgende Vernetzung notwendig ist.

Innerhalb des Herstellungsprozesses erfolgt ein gezielte Mastikation zur Einstellung der Produkteigenschaften wie Kohäsion und Adhäsion der Selbstklebemasse. Eine aufwendige vorgeschaltet Mastikation z. B. über Walzen ist nicht notwendig. Die Herstellung erfolgt in einem kontinuierlichen Arbeitsschritt.

Gelöst wird diese Aufgabe durch ein Verfahren, wie es im Anspruch 1 dargelegt ist. Gegenstand der Unteransprüche sind dabei vorteilhafte Weiterbildungen der Verfahren.

Demgemäß betrifft die Erfindung ein Verfahren zur kontinuierlichen lösungsmittelfreien Herstellung von Selbstklebemassen auf Basis von Naturkautschuk mit mindestens einem pharmazeutischen Wirkstoff in einem kontinuierlich arbeitenden Aggregat, welches zumindest einen Füllteil, einen Mastifikations- und Compoundierteil und einen Ausgabeteil aufweist, wobei der Mastifikations- und Compoundierteil mehrere Möglichkeiten zur Zugabe von festen und/oder flüssigen Komponenten entlang der Förderstrecke aufweist und dessen Scherbeanspruchung auf das hindurchgeförderte Gut zonal unterschiedlich ist. Hierbei ist die Zugabereinfolge und - Position der Massekomponenten und die gezielt auf die Rezeptur abgestimmte Geometrie und Temperaturführung des kontinuierlichen Herstellaggregats (Extruder) entscheidend für die Produkteigenschaften der Selbsklebemasse.

Beansprucht wird auch die als Verfahrensprodukt erhaltene wirkstoffhaltige Naturkautschukklebmasse, insbesondere zu Verwendung in medizinischen Pflastern.

Der Herstellprozess besteht aus den folgenden Schritten

a) Vorlage von Naturkautschuk, gegebenenfalls mit einem Trennhilfsmittel in den Füllteil des Aggregats, gegebenenfalls Vorlage von niedermolekularem Synthesekautschuk, Füllstoffen, Weichmachern, Tackifiern, Harzen und/oder Additiven,

b) Übergabe der Vorlagekomponenten der Selbstklebemasse aus dem Füllteil in den Mastikations- und Compoundierteil des Extruders,

c) Zugabe mindestens eines Stabilisators im Bereich des Mastikations- und Compoundierteils,

d) Zugabe von mindestens einem pharmazeutischen Wirkstoff und gegebenenfalls Zugabe der nicht in dem Füllteil aufgegebenen Komponenten der Selbstklebemasse wie niedermolekulares SBC, Füllstoffe, Weichmacher, Tackifier, Harze und/oder Additive,

e) Herstellung einer homogenen Selbstklebemasse durch eine Kombination aus Förder-Misch- und Knetelementen im Compoundierteil

f) Austragen der Selbstklebemasse.

Die Zugabe von mindestens einem pharmazeutischen Wirkstoff in den Compoundierteil und die gegebenenfalls erforderliche Zugabe der nicht in dem Füllteil aufgegebenen Komponenten der Selbstklebemasse in den Compoundierteil wie niedermolekulares SBC, Füllstoffe, Weichmacher, Tackifier, Harze und/oder Additive kann über die gesamte Länge des Compoundierteils erfolgen. Insbesondere sind mehrere Zudosierungsstellen möglich, so dass jede einzelne Komponente je nach Anforderung an die Prozessführung gezielt über einen eigenen Zulauf in fester (Granulat, Pulver) oder flüssiger (Flüssigkeit, Schmelze) Form zudosiert werden kann. Eine Zudosierung von zusätzlichem Sauerstoff erfolgt erfindungsgemäß nicht, wobei unter Sauerstoff auch sauerstoffhaltige Gasgemische zu verstehen sind.

Die kontinuierliche lösungsmittelfreie Verarbeitung von Naturkautschuken zu wirkstoffhaltigen, medizinischen Klebemassen ist technisch sehr schwierig und ist nach dem erfindungsgemäßen Verfahren erstmals ohne anschließende ESH-Vernetzung möglich.

Der Vorteil liegt in der Kombination der PSA-Rohstoffe (PSA = Pressure Sensitiv Adhesive) mit dem Wirkstoffsystem und einem Stabilisator zur Steuerung des Abbaugrades des Kautschuks im kontinuierlichen Mischprozess. Besonders wichtig ist dabei die örtliche und zeitliche Reihenfolge der Rohstoffzugabe sowie die Aufbereitung der dosierten Stoffe durch Temperatur-, Misch- und Schersteuerung im Compoundierprozess. Ausschlaggebend für die hohe Homogenität des Wirkstoffes in der Polymermatrix ist neben der exakten Dosierung, die Zugabeposition in den Mischprozess und die Dispergierung in einem Rheologie- und Temperaturfenster.

Vorteilhaft werden für das erfindungsgemäße Herstellverfahren Mischungen aus

- 10 bis 30% Natur-Kautschuk (z. B. 1,4 Polyisopren),
- 10 bis 30 % Harze (z. B. Kolophoniumharze, aliphatische und aromatische Kohlenwasserstoffharze, Terpenharze und Terpenphenolharze) in beliebiger Kombination und
- 0,001 bis 5 % Stabilisatoren
- 3 bis 15 % Weichmacher/Tackifier (z. B. Öle und Wachse),
- 0.01 bis 20 % pharmazeutische Wirkstoffe eingesetzt und gegebenenfalls bis zu
- 0 - 40 % Füllstoffe (z. B. Reismehl, Cellulose, Kreide),
- 0 - 5% Additive (z. B. Alterungsschutz, Antioxidatien) und
- 0 - 30% Styrolblockcopolymer eingearbeitet.

Der Einsatz eines kontinuierlichen Verfahrens bedeutet gegenüber dem Batch Verfahren (einzelne Masseansätze) eine deutliche Steigerung der Produktivität. Die Beschichtung auf das Trägermaterial kann inline (direkt aus dem Extruder) erfolgen, so dass zusätzliche Arbeitsschritte entfallen. Durch den Wegfall des Lösungsmittels im Herstellprozess wird dieser erheblich kostengünstiger und sicherer.

Ein wesentlicher Vorteil des erfindungsgemäßen Verfahrens ist, dass die gleich bleibend hohe Produktqualität

erreicht werden kann, da sich eine Verfahrensteuerung über einen Vergleich der Molekulargewichtsverteilung und der dynamischen Viskosität der Masse vornehmen lässt.

Dies stellt gegenüber den aus dem Stand der Technik bekannten diskontinuierlichen Verfahren einen entscheidenden Vorteil dar.

Durch die gezielte Zugabe von Stabilisatoren, kommt das erfindungsgemäße Verfahren ohne die Zudosierung von Sauerstoff aus. Dadurch ist es möglich dotierte Klebmassen herzustellen, in denen die Wirkstoffe durch Sauerstoff bei den im Extruder herrschenden Bedingungen zerstört würden. Bei der Verarbeitung von sehr empfindlichen Roh- und Wirkstoffe ist es sogar von Vorteil eine Schutzgasatmosphäre zu verwenden.

Besonders vorteilhaft am erfindungsgemäßen Verfahren ist, das keine Mastikationshilfsmittel, wie aromatische oder heterocydische Mercaptane beziehungsweise deren Zink-Salze oder Di-sulfide, eingesetzt werden müssen, die für zum Teil medizinische Klebmassen nicht zugelassen sind.

Die erfindungsgemäß hergestellten wirkstoffhaltigen Klebmassen weisen eine hervorragende medizinische Wirksamkeit auf, die sich anhand von Freisetzungsversuchen belegen lässt.

Das kontinuierliche Verfahren ist gegenüber der üblichen Batchherstellung deutlich produktiver und damit kostengünstiger (kompakte Anlage, geringer Platzbedarf, hohe Betriebssicherheit, hohe Produktivität). Da die Masse lösemittelfrei hergestellt wird, ist das Herstellverfahren deutlich vereinfacht. Es ist nicht mehr notwendig eine aufwendige, teure und große Lösemittelaufbereitungsanlage zu betreiben. Außerdem müssen keine Richtlinien zum Explosionsschutz beachtet werden. Die Anlage benötigt außerdem keine Trockenkanäle und kann deutlich schneller betrieben werden.

Der Vorteil des Einsatzes von Naturkautschuk als Gerüstpolymer in der Selbstklebemasse besteht darin, dass über den Herstellungsprozess die Klebeigenschaften wie Kohäsion und Scherfestigkeit gezielt eingestellt werden können. Somit können die Produkteigenschaften besser auf den Verbraucher zugeschnitten werden. Naturkautschukhaltige Selbstklebemassen zur Anwendung auf der Haut zeichnen sich durch sehr starke weiche Klebeigenschaften mit sehr hoher Anfangsklebrigkeit (Tack) aus. Auch auf feuchter Haut ist eine gute Verklebung gegeben. Damit ist eine zuverlässige Verklebung gewährleistet, die beim Einsatz eines transdermalen Pflasters zur Sicherstellung der Wirkstofffreisetzung zwingend notwendig ist.

Der Vorteil gegenüber dem Stand der Technik liegt in der Kombination der Naturkautschuk-Rohstoffe mit einem Stabilisator zu Beginn des kontinuierlichen Mastikations- und Mischprozess, das heißt bereits am Anfang des Extruders bzw. dessen Einzugszone. Besonders wichtig ist dabei die örtliche und zeitliche Reihenfolge der Roh- und Wirkstoffzugabe sowie die Aufbereitung der dosierten Stoffe durch Temperatur-, Misch- und Schersteuerung im Compoundierprozess. Ausschlaggebend für die hohe Homogenität des Wirkstoffes in der Polymermatrix ist neben der exakten Dosierung, der Eintrag in den Mischprozess und die Dispergierung in einem engen Rheologie- und Temperaturfenster.

Die Erfindung wird nachfolgend unter Bezugnahme auf ein in den Zeichnungen dargestellten Ausführungsbeispiel näher erläutert.

Es zeigt:

Abbildung 1: Schematische Darstellung des erfindungsgemäßen kontinuierlichen Produktionsverfahrens

Abbildung 2: Beispiel für eine vorteilhafte Schneckengeometrie, mit GFA = Förderschnecke, KB = Knetblock, KS = Knetscheibe, GFM = Mischblock, Angaben mit Gewindezahl, Steigung [°], Länge [mm] und Drehrichtung.

Abbildung 3: Schematische Darstellung des Beschichtungsprozesses.

Als besonders vorteilhaft hat sich die Verwendung eines Doppelschneckenextruders mit mindestens einer Dosieröffnung, vorzugsweise zwischen zwei und 12, und mindestens einer Entgasungsöffnung als kontinuierlich arbeitendes Aggregat herausgestellt. Der Doppelschneckenextruder ermöglicht kurze Mischzeiten und damit eine besonders schonende Verarbeitung der Masse, insbesondere thermisch sensibler Einsatzkomponenten.

Die segmentierte Extruderschnecke bietet beliebige Kombinationsmöglichkeiten und Abfolgen von unterschiedlich starken Knetelementen, Mischaggregaten und Förderschnecken mit unterschiedlicher Förderwirkung.

Die Mastikation ist zu Beginn des Extrusionsvorganges am stärksten und schwächt sich mit zunehmender Verweitdauer bzw. Verfahrensweg (Durchwanderungsweg) im Extruder ab. Um jedoch ein gleichmäßiges Produkt zu erhalten, muss die Mastifikation durch Zugabe von mindestens einem Stabilisator zu Beginn des Mastikationsvorganges begrenzt werden.

Die eingesetzten Stabilisatoren wirken als Radikalfänger und begrenzen den Abbau des Naturkautschuks, so dass bevorzugt Anteile mit einem Molgewicht über 1 E6,5 g/mol abgebaut werden und Polymereinheiten mit einem Molgewicht zwischen 1 E2 und 1E6,5 g/mol entstehen. Dazu wird der Extruder zonenweise auf unterschiedlichen Temperaturen gehalten, wobei die Temperatur zumindest im ersten Abschnitten des Extruders höher ist als die Temperatur im letzten Abschnitt. Neben der Temperaturführung von hohen auf niedrigere Temperaturen, ist es entscheidend, dass die Viskosität während des Mastikationsprozesses durch Zugabe von Viskositätserniedrigenden Zusätzen herabgesetzt

wird. Dies bewirkt eine über den gesamten Prozess kontinuierliche schonende Verarbeitung der Rohstoffe, insbesondere der Wirkstoffe.

Die Weichmacher werden bevorzugt erst in den letzten Extrusionsschritten zudosiert, da diese eine sehr starke viskositätserniedriegende Wirkung haben und nur eine homogene Verteilung in der Hotmeltmasse erfahren müssen.

Die Zuführung von Wirkstoffen erfolgt erfindungsgemäß ebenfalls in einer der letzen Extruderzonen um die thermische und mechanische Belastung gering zu halten. Die Verweilzeit des Wirkstoffes im Verfahrensteil ist damit extrem kurz (vorzugsweise < 1 min).

Bei geringeren Viskositäten erfolgt ein niedrigerer Energieeintrag und damit eine geringere Mastikation, was zum Abbau von sehr hohen Molekulargewichten führt, jedoch die bereits vorliegenden Polymereinheiten mit niedrigen Molekulargewichten nicht weiter degradiert. Somit wird eine sehr homogene Molmassenverteilung im Bereich von 1E2 und 1E6,5 g/mol erhalten.

Dies ist auch daraus ersichtlich, dass das ausgegebene Produkt homogen ist und keinerlei Stippen (nicht aufgeschlossenen/eingearbeiteten Kautschukpartikel) aufweist.

Im Aggregat sollte am Anfang eine Temperatur von 100 bis 220°C und am Ende eine Temperatur von bevorzugt 65 bis 110° C eingehalten werden, um eine thermische Schädigung insbesondere des oder der Wirkstoffe auszuschließen.

Der Abbau des Kautschuks erfolgt vornehmlich im ersten Drittel des Extruders bei höheren Temperaturen. Die Einarbeitung von Wirkstoffen und verdünnenden Ölen, wie zum Beispiel Gelböl und Wollwachs, erfolgt im letzten Drittel des Extruders bei niedrigeren Temperaturen, denn in diesem Bereich ist die Verarbeitung (Mastikation) des Kautschuks bereits abgeschlossen.

Im zweiten, vorteilhafterweise im Verbund mit dem Compoundierschritt im Doppelschneckenextruder erfolgenden Verfahrensschritt wird die erfindungsgemäß hergestellte Selbstklebemasse mit einem Auftragswerk auf einen bahnförmigen Träger, auf eine Trennfolie oder auf ein Trennpapier lösemittelfrei beschichtet. Die Beschichtung kann vollflächig, aber auch partiell erfolgen.

Um einen definierten, luftblasenfreien Masseauftrag auf dem bahnförmigen Material zu erhalten, ist es vorteilhaft, dass die Selbstklebemasse vor Eintritt in die Beschichtungseinheit einer Entgasung unterworfen wird, was besonders wichtig ist im Falle der Verwendung von Schutzgasen während des Compoundierprozesses im Doppelschneckenextruder.

Gemäß des Verfahrens der vorliegenden Erfindung kann eine Entgasung unter Einfluss von Unterdruck oder gegen die Atmosphäre vorzugsweise in Schneckenmaschinen erfolgen.

Zur Beschichtung auf bahnförmige Materialien eignen sich verschiedene Verfahren. Lösungsmittelfreie Selbstklebemassen lassen sich mittels einer dem Doppelschneckenextruder nachgeschalteten Extrusionsdüse beschichten. Zum Druckaufbau für die Düsenbeschichtung werden Einschneckenextruder und/oder Schmelzepumpen besonders bevorzugt, so dass die Beschichtung der bahnförmigen Trägermaterialien mit Masseaufträgen sehr geringer Schwankungsbreite erfolgen kann.

Eine weitere Möglichkeit zur Beschichtung von bahnförmigen Trägermaterialien mit der nach erfindungsgemäßen Verfahren hergestellten wirkstoffhaltigen Selbstklebemasse ist die Verwendung von Walzenbeschichtungsauftragswerken oder Mehrwalzen-Beschichtungskalandern, die vorzugsweise aus mindestens zwei Beschichtungswalzen bestehen, wobei die Selbstklebemasse bei Durchgang durch einen oder mehrere Walzenspalte vor Übergabe auf das bahnförmige Material auf die gewünschte Dicke geformt wird. Dieses Beschichtungsverfahren wird besonders dann bevorzugt, wenn eine Beschichtung mit Extrusionsdüsen allein nicht mehr die erforderte Genauigkeit im Masseauftrag liefert.

Je nach Art des zu beschichtenden bahnförmigen Trägermaterials kann die Beschichtung im Gleichlauf- oder Gegenlaufverfahren erfolgen.

Die Beschichtung ist auf Walzenbeschichtungsauftragswerken oder Mehrwalzen-Beschichtungskalandern bei Temperaturen unterhalb von 120 °C möglich, so dass auch Selbstklebemassen beschichtet werden können, die thermisch empfindliche Wirkstoffe enthalten. Zum Zwecke erhöhter Gasblasenfreiheit der beschichteten Klebmasse kann zwischen Doppelschneckenextruder und Auftragswerk eine Vakuumentgasung, zum Beispiel eine Vakuumkammer, ein Entgasungsextruder, Luftrakel oder ähnliches installiert sein.

Gemäß des Verfahrens vorliegender Erfindung erfolgt keine eigenschaftsschädigende Mastikation des Naturkautschuks, da kurz nach dessen Vorlage die Stabilisatorzugabe und damit eine Regelung des Mastikationsprozesses erfolgt. Zusätzlich wird der Mastikationsprozess über die Viskosität des Compounds mittels Zugabe weiterer Massekomponenten (z. B. Harze) gesteuert

Der Doppelschneckenextruder sollte über einen oder vorzugsweise mehrere separate Temperier- beziehungsweise Kühlkreise verfügen, um ein Temperaturregime zu ermöglichen, das den Einsatz thermisch empfindlicher pharmazeutischer Wirkstoffe erlaubt. In Fällen, wo dies nicht erforderlich ist, können die Temperierkreise auch miteinander verbunden werden, um die Anzahl an Temperiergeräten möglichst gering zu halten.

Abbildung 2 zeigt schematisch den Aufbau eines erfindungsgemäß verwendbaren Extruders mit 13 Zylindern,

wobei die Art der Schneckengeometrie und die Reihenfolge der Seitenbeschickung ersichtlich ist. Die Schneckengeometrie ist für jeden Zylinder angegeben, dabei bedeuten: GFA = Förderschnecke, KB = Knetblock, KS = Knetscheibe, GFM = Mischblock. Die Zahlenfolge gibt die Gewindezahl, die Steigung in ° und die Länge in mm sowie die Drehrichtung des Schneckenelements wieder.

Die Dosierreihenfolge und Menge ist so abgestimmt, dass der gewünschte Abbaugrad des Kautschuks erreicht wird. Der Abbaugrad bestimmt die Eigenschaften der Masse. Bei einem hohen Abbaugrad entsteht eine stark klebende Masse. Mit einem niedrigen Abbaugrad erhält man eine Masse mit hoher Kohäsion und Scherfestigkeit. Der Abbaugrad wird über die dynamische Viskosität, den K-Wert und die Molekulargewichtsverteilung eingestellt und kontrolliert.

Ein schonender Abbau wird im Besonderen dadurch erreicht, dass eine Menge von 10 bis 80 % des Harzes bereits mit dem Kautschuk gemeinsam am Anfang des Herstellprozesses zudosiert wird.

Neben der Zugabereihenfolge wird der Energieeintrag beim Compoundierprozess durch die gezielte Auswahl und Abfolge von Misch-, Knet- und Förderelementen im Doppelschneckenextruder definiert. Die Schneckengeometrie (Beispiel s. Abbildung 2) ist hierbei speziell auf die Rezeptur abgestimmt. Neben der Schneckengeometrie wird der Compoundierprozess über die Temperaturführung im Verfahrensteil eingestellt.

Erfindungsgemäß beträgt die Temperatur im Extruder je nach Zone zwischen 65 und 220 °C. Innerhalb des Extruders wird dabei ein Temperaturprofil ausgebildet, wobei hohe Temperaturen zur Verarbeitung von Kautschuk und Harz (150 - 220°C), niedrige emperaturen zur Einarbeitung des Wirkstoffes (65 - -100°C) benötigt werden.

Erfindungsgemäß beträgt der Druck im Austragsbereich zwischen 30 und 80 bar. Der Energieeintrag wird außerdem über die Schneckendrehzahl beeinflusst, der nötige Energieeintrag wird erfindungsgemäß bei einer Schnekkendrehzahl von 100 bis 600 U/min erreicht, insbesondere Drehzahlen zwischen 200 und 400 U/min sind vorteilhaft.

Nach dem Compoundieren wird die Masse inline über eine Zahnradpumpe durch eine Extrusionsdüse aus dem Doppelschneckenextruder in die Beschichtungsstation gefördert (s. Abbildung 3).

Die Masse wird im Walzenspalt auf einen Masseauftrag von 100-500 g/$m^2$ ausgezogen. Die obere Walze führt den Träger und die untere Walze läuft mit 1 -10 % der Bahngeschwindigkeit. Durch die Differenzgeschwindigkeit entstehen große Scherkräfte im Spalt, die das Beschichten mit den genannten Masseaufträgen ermöglichen. Je nach Massesystem werden die Walzen auf Temperaturen von 20-100 °C beheizt oder gekühlt.

In einem nachfolgenden Kaschierwerk mit zwei Walzen wird der Träger auf die Masse kaschiert. Über Druck und Temperatur werden Masseverankerung und Eindringtiefe in den Träger eingestellt.

Das erfindungsgemäße Verfahren erlaubt die Herstellung von Selbstklebemassen mit pharmazeutischen Wirkstoffen und insbesondere im Verbund mit einer nachgeschalteten Beschichtungseinheit die Herstellung von selbstklebend ausgerüsteten Artikeln, die ihrerseits zur Herstellung von Pflastern oder Binden eingesetzt werden, unter Erlangung besonderer Kostenvorteile.

Das Verfahren besteht im wesentlichen aus den bereits dargelegten Verfahrensschritten, welche optional unter einer Schutzgasatmosphäre zur Vermeidung von oxidativem Polymerabbau durchgeführt werden können.

Im Compoundierungsschritt wird eine Klebmasse aus Naturkautschuk, einem oder mehreren pharmazeutischen Wirkstoffen und den benötigten Zuschlagstoffen wie niedermolekulares SBC, Füllstoffe, Weichmacher, Tackifier, Harze und/oder Additive bevorzugt in einem Doppelschneckenextruder lösungsmittelfrei hergestellt. Die pharmazeutischen Wirkstoffe können dabei unmittelbar zu Beginn der Compoundierung zugesetzt werden, können aber auch -je nach Empfindlichkeit des Wirkstoffes - erst zu einem späteren Zeitpunkt in den Doppelschneckenextruder gegeben werden. Die Zugabe kann in reiner oder gelöster Form in flüssigem oder festem Zustand erfolgen.

Erfindungsgemäß basiert die Heißschmelzselbstklebemasse, in welcher mindestens ein pharmazeutischer Wirkstoff eingearbeitet wird, aus

- Naturkautschuk bzw. Polyisoprene,
- Stabilisatoren
- Klebrig machenden Harzen,
- Tackifiern,
- Weichmachern,
- Füllstoffen, Styrolblockcopolymere und/oder Additiven.

Als pharmazeutische Wirkstoffe werden Substanzen verstanden, die in menschlichen oder tierischen Organismen zur Verhütung, Heilung, Linderung oder Erkennung von Krankheiten dienen. Die eingesetzten pharmazeutischen Wirkstoffe können sowohl systemisch als auch lokal wirksam sein.

(z. B. Salizylsäure, Capsaicin, Ibuprofen)

Typische, erfindungsgemäß eingesetzte Wirkstoffe sind hierbei:

Aceclidin, Amfetaminil, Amfetamin, Amylnitrit, Apophedrin, Atebrin, Alpostadil, Azulen, Arecolin, Anethol, Amylenhydrat, Acetylcholin, Acridin, Adenosintriphosphorsäure, Äpfelsäure, Alimemazin, Allithiamin, Aminoethanol, Apyzin, Apiol, Azatadin, Alprenolol, Äthinazon, Bisabolol, Bisnorephedrin, Butacetoluid, Benactyzin, Campher, Colecalciferol, Chloralhydrat, Clemastin, Chlorobutanol, Capsaicin, Cyclopentamin, Clobutinol, Chamazulen, Dimethocain, Codein, Chloropromazin, Chinin, Chlorthymol, Cyclophosphamid, Cinchocain, Chlorambuzil, Chlorphenesin, Dexchlorpheniramin, Dinoproston, Dixyrazin, Ephedrin, Ethosuximid, Enallylpropymal, Emylcamat, Erytroltetranitrat, Emetin, Eucalyptol, Etofenamat, Ethylmorphin, Fentanyl, Fluanison, Guajazulen, Hyoscyamin, Histamin, Fencarbamid, Hydroxycain, Hexylresorcin, Isoaminilcitrat, Isosorbiddinitrat, Ibuprofen, Jod, Jodoform, Isoaminil, Lidocain, Lopirin, Levamisol, Methadon, Methyprylon, Methylphenidat, Mephenesin, Methylephedrin, Meclastin, Methopromazin, Mesuximid, Menthol, Methylpentinol, Metixen, Mesoprostol, Nicethamid, Norpseudoephedrin, Nonylsäurevanillyamid, Oxytetracain, Oxyprenolol, Oxyphenbutazon, Oxychinolin, Pinen, Prolintan, Procyclidin, Piperazin, Pivazid, Phensuximid, Procain, Phenindamin, Promethazin, Pentetrazol, Profenamin, Perazin, Phenol, Pethidin, Pilocarpin, Prenylamin, Phenoxybenzamin, Resochin, Scopolamin, Salicylsäure, Spartein, Timolol, Trifluperazin, Tetracain, Trimipramin, Tranylcypromin, Trimethadion, Tybamat, Thymol, Thioridazin, Valproinsäure, Verapamil, sowie weitere, dem Fachmann geläufige, über die Haut, eingeschlossen Schleimhäute, aufnehmbare Wirkstoffe. Selbstverständlich ist diese Aufzählung nicht abschließend.

Besonders bedeutende Wirkstoffe sollen im folgenden näher aufgezählt und klassifiziert werden - ohne auch hier den Anspruch der Vollständigkeit im Rahmen der vorliegenden Erfindung zu erheben:

| Indikation: | Wirkstoff |
|---|---|
| Antimykotika | Naftifin: ((E)-N-Cinnamyl-N-methyl-1-naphthalinmethanamin)<br>Amorolfin: ((±)-cis-2,6-Dimethyl-4-[2-methyl-3-(4-tert- pentylphenyl)-propyl] morpholin)<br>Tolnaftat: (0-(2-Naphthyl)-N-methyl-N-m-tolyl-thiocarbamat)<br>Clotrimazol: (1-[(2-Chlorphenyl)-diphenylmethyl]-1H-imidazol) |
| Antiseptika | Triclosan<br>Ethacridin<br>Chlorhexidin<br>Hexetidin<br>Dodicin<br>Iod |
| Nichtsteroidale Antirheumatika | Methylsalicylat<br>Etofenamat<br>Indomethacin: ([1-(4-Chlorbenzoyl)-5-methoxy-2-methyl-1H- indol- 3-yl] essigsäure) |
| Antipuriginosa | Crotamiton |
| Lokalanästhetika | Benzocain |
| Antipsoriatika | |
| Keratolytika | Harnstoff |

Weitere, für die Wundheilung förderliche Wirkstoffe, wie Silbersulfadiazin, können ebenfalls eingesetzt werden.

Besonders vorteilhaft und im Sinne der Erfindung können auch hyperämisierende Wirkstoffe wie natürliche Wirkstoffe des Cayenne-Pfeffers oder synthetische Wirkstoffe wie Nonivamid, Nicotinsäurederivate, bevorzugt Bencylnicotinat oder Propylnicotinat, genannt werden beziehungsweise Antiphlogistika und/oder Analgetika.

Beispielhaft seien Capsaicin ( [8-Methyl-trans-6-nonensäure-(4-hydroxy-3- methoxybenzylamid)] )

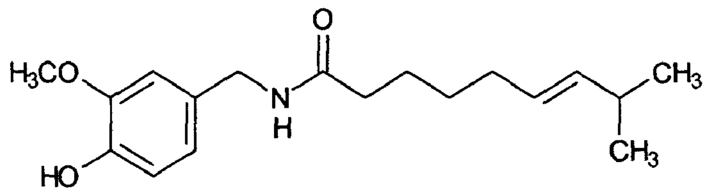

Nonivamid

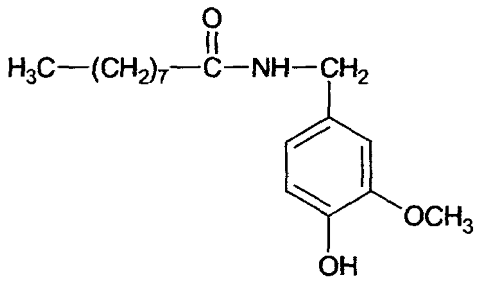

Nicotinsäurebenzylester (Benzylnicotinat)

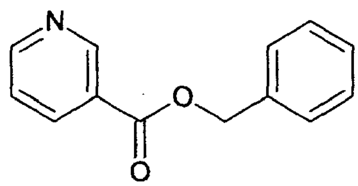

genannt.

Von besonderer Bedeutung unter den Wirkstoffen sind die Desinfektionsmittel beziehungsweise Antiseptika hervorzuheben, so dass deren Verwendung nochmals betont werden soll.

Als Desinfektionsmittel werden Stoffe bezeichnet, die zur Desinfektion, d. h., zur Bekämpfung pathogener Mikroorganismen (zum Beispiel Bakterien, Viren, Sporen, Klein- und Schimmelpilze) geeignet sind und zwar im allgemeinen durch Anwendung an der Oberfläche von Haut, Kleidung, Geräten, Räumen, aber auch von Trinkwasser, Nahrungsmitteln, Saatgut (Beizen) und als Bodendesinfektionsmittel.

Besonders lokal anzuwendende Desinfektionsmittel, zum Beispiel zur Wunddesinfektion, werden auch als Antiseptika bezeichnet.

Insbesondere sind als Antiseptikum die Milchsäure-Abkömmlinge, wie Ester sowie Oligo- und Polymilchsäure, einzusetzen.

Unter Milchsäureester sind die häufig als Lactate der jeweiligen Alkohol-Komponente benannten Ester der allgemeinen Formel

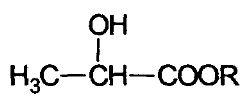

$R=CH_3$ : a
$R = C_2H_5$ : b
$R = CH(CH_3)_2$ : c

R = $(CH2)_3CH_3$: d

bekannt, die in der Mehrzahl bei 20 °C flüssige oder tiefschmelzende Produkte sind und die in Wasser, mit Ausnahme der niederen Alkylester, wenig, in Alkohol und Ether gut löslich sind.

Man unterscheidet

(a) Milchsäuremethylester (Methyllactat), $C_4H_8O_3$, $M_R$ 104,10, Siedepunkt 145 °C

(b) Milchsäureethylester (Ethyllactat), $C_5H_{10}O_3$, $M_R$ 118,13, D. 1,03, Siedepunkt 154 °C

(c) Milchsäure-isopropylester (isopropyllactat), $C_6H_{12}O_3$, $M_R$ 132,15, D. 0,9980, Siedepunkt 167 °C

(d) Milchsäurebutylester (Butyllactat), $C_7H_{14}0_3$, $M_R$ 146,18, D. 0,9803, Siedepunkt 187 °C

Polymilchsäure (Polylactid) ist ein Polyester auf Basis von Milchsäure, aus deren Lactid sie durch Ringöffnungspolymerisation hergestellt werden kann.

Der Wirkstoffanteil in der Selbstklebemasse liegt vorzugsweise zwischen 0,001 Gew.-% und 10 Gew.-%, bevorzugt zwischen 0,01 Gew.-% und 7 Gew.-%, besonders bevorzugt zwischen 0,03 Gew.-% und 5 Gew.-%.

Unter natürlichem Kautschuk bzw. Naturkautschuk im Sinne der Erfindung sind Naturkautschuk, gereinigter bzw. aufbereiteter Naturkautschuk zu verstehen. Insbesondere erfindungsgemäß sind z. B. Crepe oder Kautschuk V145 mit einem K-Wert zwischen 130 und 170.

Der auch als Eigenviskosität bezeichnete K-Wert (Fikentscher-Konstante) ist ein über Viskositätsmessungen von Polymer-Lösungen einfach zu bestimmender und daher im technischen Bereich häufig benützter Parameter zur Charakterisierung von Polymeren. Für eine bestimmte Polymer-Sorte wird er unter standardisierten Messbedingungen als alleine abhängig von der mittleren Molmasse der untersuchten Probe angenommen und über die Beziehung K-Wert = 1000 k nach der Fikentscher-Gleichung

$$k = \frac{1{,}5 \lg \eta_r - 1 \pm \sqrt{1 + \left(\frac{2}{c} + 2 + 1{,}5 \lg \eta_r\right) \cdot 1{,}5 \lg \eta_r}}{150 + 300c}$$

berechnet, in der bedeuten: $\eta_r$ = relative Viskosität (dynamische Viskosität der Lösung/dynamischer Viskosität des Lösungsmittels.) und c = Massenkonzentration. an Polymer in der Lösung (in $g/cm^3$).

Erfindungsgemäße Stabilisatoren sind Verbindungen die die beim Kettenabbau entstehende Radikale binden und damit wird verhindern, dass die Radikale weitere Abbaureaktionen auslösen.

Besonders vorteilhaft sind 2,2-Thiodiethylbis-(3-,5-di-tert.-butyl-4-hydroxyphenyl)-propionat und 2,2'-Methylen-bis-(6- tert.-butyl-p-cresol) und deren Handelsformen:

Lowinox® 22 M 46 2,2'-Methylen-bis-(6- tert.-butyl-p-cresol) (97%ig)

Irganox® 1035 2,2-Thiodiethylbis-(3-,5-di-tert.-butyl-4-hydroxyphenyl)-propionat

Irganox 1035 ist ein schwefelhaltiges, phenolisches Antioxidans, das organische Polymere wirksam gegen thermische Oxidation stabilisiert.

Der Anteil an Stabilisatoren liegt zwischen 0,001 Gew.-% und 5 Gew.-%, bevorzugt zwischen 0,01 Gew.-% und 3 Gew.-%.

Als klebrigmachende Harze sind ausnahmslos alle vorbekannten und in der Literatur beschriebenen Klebharze einsetzbar. Genannt seien stellvertretend die Kolophoniumharze, deren disproportionierte, hydrierte, polymerisierte, veresterte Derivate und Salze, die aliphatischen und aromatischen Kohlenwasserstoffharze, Terpenharze und Terpenphenolharze. Beliebige Kombinationen dieser und weiterer Harze können eingesetzt werden, um die Eigenschaften der resultierenden Klebmasse wunschgemäß einzustellen. Auf die Darstellung des Wissensstandes im "Handbook of Pressure Sensitive Adhesive Technology" von Donatas Satas (van Nostrand, 1999) sei ausdrücklich hingewiesen.

Der Harzanteil liegt insbesondere zwischen 0 Gew.-% und 80 Gew.-%, bevorzugt zwischen 5 Gew.-% und 60 Gew.-%, besonders bevorzugt zwischen 10 Gew.-% und 50 Gew.-%.

Kohlenwasserstoffharz ist eine Sammelbezeichnung für thermoplastische, farblose bis intensiv braun gefärbte Polymere mit einer Molmasse von im allgemeinen unter 2000 g/mol.

Sie lassen sich nach ihrer Provenienz in drei große Gruppen einteilen: In Petroleum-, Kohlenteer- und Terpenharze. Die wichtigsten Kohlenteerharze sind die Cumaron-Inden-Harze. Die Kohlenwasserstoffharze werden durch Polymerisation der aus den Rohstoffen isolierbaren ungesättigten Verbindungen gewonnen.

Zu den Kohlenwasserstoffharzen werden auch durch Polymerisation von Monomeren wie Styrol bzw. durch Polykondensationen (bestimmte Formaldehyd-Harze) zugängliche Polymere mit entsprechend niedriger Molmasse gerechnet. Kohlenwasserstoffharze sind Produkte mit in weiten Grenzen von kleiner 0 °C (bei 20 °C flüssige Kohlenwasserstoffharze) bis über 200 °C variierendem Erweichungsbereich und einer Dichte von ca. 0,9 bis 1,2 g/cm$^3$.

Sie sind löslich in organischen Lösungsmitteln wie Ethern, Estern, Ketonen und chlorierten Kohlenwasserstoffen, unlöslich in Alkoholen und Wasser.

Unter Kolophoniumharz wird ein natürliches Harz verstanden, das aus dem Rohharz von Koniferen gewonnen wird. Man unterscheidet drei Kolophonium-Typen: Balsamharz als Destillationsrückstand von Terpentinöl, Wurzelharz als Extrakt von Koniferen-Wurzelstöcken und Tallharz, der Destillationsrückstand von Tallöl. Die mengenmäßig größte Bedeutung hat Balsamharz.

Kolophonium ist ein sprödes, transparentes Produkt von roter bis brauner Farbe. Es ist wasserunlöslich, löslich dagegen in vielen organischen Lösungsmitteln wie (chlorierten) aliphatischen und aromatischen Kohlenwasserstoffen, Estern, Ethern und Ketonen sowie in pflanzlichen und mineralischen Ölen. Der Erweichungspunkt von Kolophonium liegt im Bereich von ca. 70 bis 80 °C.

Kolophonium ist ein Gemisch aus ca. 90 % Harzsäuren und 10 % Neutral-Stoffen (Fettsäureester, Terpenalkohole und Kohlenwasserstoffe). Die wichtigsten Kolophonium-Harzsäuren sind ungesättigte Carbonsäuren der Bruttoformel $C_{20}H_{30}O_2$, Abietin-, Neoabietin-, Lävopimar-, Pimar-, Isopimar-, und Palustrinsäure, neben hydrierter und dehydrierter Abietinsäure.

Die Mengenverhältnisse dieser Säuren variieren in Abhängigkeit von der Provenienz des Kolophoniums.

Als Tackifier kommen alle bekannten klebrigmachenden Polymere in Frage, z. B. aus der Gruppe der Polyisoprene und Polybutadiene. Der Tackifieranteil liegt insbesondere zwischen 0 Gew.-% und 50 Gew.-%, bevorzugt zwischen 0 Gew.-% und 40 Gew.-%, besonders bevorzugt zwischen 0 Gew.-% und 30 Gew.-%.

Als Weichmacher können alle bekannten weichmachenden Substanzen sowie pharmazeutische Hilfsstoffe eingesetzt werden. Dazu zählen unter anderem die paraffinischen und naphthenischen Öle, (funktionalisierte) Oligomere wie Oligobutadiene, -isoprene, flüssige Nitrilkautschuke, flüssige Terpenharze, pflanzliche und tierische Öle und Fette, Fettsäureester, Phthalate, Alkohole, funktionalisierte Acrylate.

Der Weichmacheranteil liegt insbesondere zwischen 0 Gew.-% und 60 Gew.-%, bevorzugt zwischen 2 Gew.-% und 40 Gew.-%.

Als Füllstoffe kommen rieselfähige Schüttgüter sowie deren Mischungen in Frage, wie zum Beispiel Cellulose, Kieselsäure, Alginate, Pektine, die nicht in der Klebmatrix löslich sind. Der Füllstoffanteil liegt insbesondere zwischen 0 Gew.-% und 60 Gew.-%, bevorzugt zwischen 0 Gew.-% und 40 Gew.-%, besonders bevorzugt zwischen 0 Gew.-% und 30 Gew.-%.

Als Styrolblockcopolymere kommen bevorzugt A-B- und/oder A-B-A- Blockcopolymere oder deren Mischungen in Frage. Die harte domänenbildende Phase A besteht vornehmlich aus Polystyrol oder dessen Derivaten. Die weiche Phase wird vornehmlich aus Polyisopren und Polybutadien oder deren Mischungen gebildet. Die phasenseparierende Struktur der Styrolblockcopolymere verhilft den Polymeren zu einem thermoplastischen Verhalten, welches sich von Polymeren mit statistisch verteilten Monomeren unterscheidet und eine mastikationsfreie Verarbeitung gewährleistet. Aufgrund der Unverträglichkeit der A- und B-Blöcke besitzen die Blockcopolymere zwei Glasübergangstemperaturen: Durch die B-Blöcke eine niedrige unterhalb der Raumtemperatur und durch die Styrolblöcke eine hohe oberhalb Raumtemperatur. Im Temperaturbereich zwischen den beiden Glasübergangstemperaturen zeigen die Blockcopolymere einerseits elastisches Verhalten aufgrund der B-Blöcke, andererseits aber bleibt der Kautschuk durch die harten Styroldomänen, die durch Nebenvalenzkräfte der Styrolblöcke entstehen, kohäsiv. Der Anteil an den Styrolblockcopolymeren liegt zwischen 5 und 90 Gew.-%, bevorzugt zwischen 10 und 85 Gew.-%.

Als Additive können Alterungsschutzmittel oder Stabilisatoren verwendet werden. Dazu zählen z.B. sterisch gehinderte Phenole, hydrolysestabile Phosphite und schwefelorganische Verbindungen. Der Additivanteil liegt zwischen 0 Gew.-% und 5 Gew.-%, bevorzugt zwischen 0 Gew.-% und 3 Gew.-%.

Als bahnförmige Trägermaterialien für die erfindungsgemäß hergestellten und verarbeiteten Selbstklebemassen sind je nach Verwendungszweck alle bekannten Träger, gegebenenfalls mit entsprechender chemischer oder physikalischer Oberflächenvorbehandlung der Streichseite sowie antiadhäsiver physikalischer Behandlung oder Beschichtung der Rückseite geeignet. Genannt seien beispielsweise gekreppte und ungekreppte Papiere, Polyethylen-, Polypropylen-, mono- oder biaxial orientierte Polypropylenfolien, Polyester-, PVC- und andere Folien, bahnförmige Schaumstoffe, beispielsweise aus Polyethylen und Polyurethan.

Beispielsweise ist auch ein metallocen-Polyethylen-Vliesstoff geeignet.

Der metallocen-Polyethylen-Viiesstoff weist vorzugsweise folgende Eigenschaften auf:

- ein Flächengewicht von 40 bis 200 g/m$^2$, insbesondere von 60 bis 120 g/m$^2$, und/oder
- eine Dicke von 0,1 bis 0,6 mm, insbesondere von 0,2 bis 0,5, und/oder
- eine Höchstzugkraft-Dehnung längs von 400 bis 700% und/oder
- eine Höchstzugkraft-Dehnung quer von 250 bis 550%.

Als Trägermaterial können weiterhin alle bekannten textilen Träger wie Gewebe, Gewirke oder Vliese verwendet werden, wobei unter "Vlies" zumindest textile Flächengebilde gemäß EN 29092 (1988) sowie Nähwirkvliese und ähnliche Systeme zu verstehen sind.

Ebenfalls können Abstandsgewebe und -gewirke mit Kaschierung verwendet werden.

Derartige Abstandsgewebe werden in der EP 0 071 212 B1 offenbart. Abstandsgewebe sind mattenförmige Schichtkörper mit einer Deckschicht aus einem Faser- oder Filamentvlies, einer Unterlagsschicht und zwischen diesen Schichten vorhandene einzelne oder Büschel von Haltefasern, die über die Fläche des Schichtkörpers verteilt durch die Partikelschicht hindurchgenadelt sind und die Deckschicht und die Unterlagsschicht untereinander verbinden. Als zusätzliches, aber nicht erforderliches Merkmal sind gemäß EP 0 071 212 B1 in den Haltefasern Partikel aus inerten Gesteinspartikeln, wie zum Beispiel Sand, Kies oder dergleichen, vorhanden.

Die durch die Partikelschicht hindurchgenadelten Haltefasern halten die Deckschicht und die Unterlagsschicht in einem Abstand voneinander und sie sind mit der Deckschicht und der Unterlagsschicht verbunden.

Abstandsgewebe oder -gewirke sind u. a. in zwei Artikeln beschrieben, und zwar einem Artikel aus der Fachzeitschrift "kettenwirk-praxis 3/93", 1993, Seiten 59 bis 63
"Raschelgewirkte Abstandsgewirke"
und einem Artikel aus der Fachzeitschrift "kettenwirk-praxis 1/94", 1994, Seiten 73 bis 76
"Raschelgewirkte Abstandsgewirke"
auf deren Inhalt hiermit Bezug genommen wird und deren Inhalt Teil dieser Offenbarung und Erfindung wird.

Als Vliesstoffe kommen besonders verfestigte Stapelfaservliese, jedoch auch Filament-, Meltblown- sowie Spinnvliese in Frage, die meist zusätzlich zu verfestigen sind. Als mögliche Verfestigungsmethoden sind für Vliese die mechanische, die thermische sowie die chemische Verfestigung bekannt. Werden bei mechanischen Verfestigungen die Fasern meist durch Verwirbelung der Einzelfasern, durch Vermaschung von Faserbündeln oder durch Einnähen von zusätzlichen Fäden rein mechanisch zusammengehalten, so lassen sich durch thermische als auch durch chemische Verfahren adhäsive (mit Bindemittel) oder kohäsive (bindemittelfrei) Faser-Faser-Bindungen erzielen. Diese lassen sich bei geeigneter Rezeptierung und Prozessführung ausschließlich oder zumindest überwiegend auf Faserknotenpunkte beschränken, so dass unter Erhalt der lockeren, offenen Struktur im Vlies trotzdem ein stabiles, dreidimensionales Netzwerk gebildet wird.

Besonders vorteilhaft haben sich Vliese erwiesen, die insbesondere durch ein Übernähen mit separaten Fäden oder durch ein Vermaschen verfestigt sind.

Derartige verfestigte Vliese werden beispielsweise auf Nähwirkmaschinen des Typs "Malivlies" der Firma Karl Meyer, ehemals Malimo, hergestellt und sind unter anderem bei den Firmen Naue Fasertechnik und Techtex GmbH beziehbar. Ein Malivlies ist dadurch gekennzeichnet, dass ein Querfaservlies durch die Bildung von Maschen aus Fasern des Vlieses verfestigt wird.

Als Träger kann weiterhin ein Vlies vom Typ Kunitvlies oder Multiknitvlies verwendet werden. Ein Kunitvlies ist dadurch gekennzeichnet, dass es aus der Verarbeitung eines längsorientierten Faservlieses zu einem Flächengebilde hervorgeht, das auf einer Seite Maschen und auf der anderen Maschenstege oder Polfaser-Falten aufweist, aber weder Fäden noch vorgefertigte Flächengebilde besitzt. Auch ein derartiges Vlies wird beispielsweise auf Nähwirkmaschinen des Typs "Kunitvlies" der Firma Karl Mayer schon seit längerer Zeit hergestellt. Ein weiteres kennzeichnendes Merkmal dieses Vlieses besteht darin, dass es als Längsfaservlies in Längsrichtung hohe Zugkräfte aufnehmen kann. Ein Multiknitvlies ist gegenüber dem Kunitvlies dadurch gekennzeichnet, dass das Vlies durch das beidseitige Durchstechen mit Nadeln sowohl auf der Ober- als auch auf der Unterseite eine Verfestigung erfährt.

Schließlich sind auch Nähvliese als Vorprodukt geeignet, ein erfindungsgemäßes Klebeband zu bilden. Ein Nähvlies wird aus einem Vliesmaterial mit einer Vielzahl parallel zueinander verlaufender Nähte gebildet. Diese Nähte entstehen durch das Einnähen oder Nähwirken von durchgehenden textilen Fäden. Für diesen Typ Vlies sind Nähwirkmaschinen des Typs "Maliwatt" der Firma Karl Mayer, ehemals Malimo, bekannt.

Weiterhin besonders vorteilhaft ist ein Stapelfaservlies, das im ersten Schritt durch mechanische Bearbeitung vorverfestigt wird oder das ein Nassvlies ist, das hydrodynamisch gelegt wurde, wobei zwischen 2% und 50% der Fasern des Vlieses Schmelzfasern sind, insbesondere zwischen 5% und 40% der Fasern des Vlieses.

Ein derartiges Vlies ist dadurch gekennzeichnet, dass die Fasern nass gelegt werden oder zum Beispiel ein Stapelfaservlies durch die Bildung von Maschen aus Fasern des Vlieses oder durch Nadelung, Vernähung beziehungsweise Luft- und/oder Wasserstrahlbearbeitung vorverfestigt wird.

In einem zweiten Schritt erfolgt die Thermofixierung, wobei die Festigkeit des Vlieses durch das Auf- oder Anschmelzen der Schmelzfasem nochmals erhöht wird.

Die Verfestigung des Vliesträgers lässt sich auch ohne Bindemittel beispielsweise durch Heißprägen mit strukturierten Walzen erreichen, wobei über Druck, Temperatur, Verweilzeit und die Prägegeometrie Eigenschaften wie Festigkeit, Dicke, Dichte, Flexibilität u. ä. gesteuert werden können.

Für die erfindungsgemäße Nutzung von Vliesen ist besonders die adhäsive Verfestigung von mechanisch vorverfestigten oder nassgelegten Vliesen von Interesse, wobei diese über Zugabe von Bindemittel in fester, flüssiger, geschäumter oder pastöser Form erfolgen kann. Prinzipielle Darreichungsformen sind vielfältig möglich, zum Beispiel feste Bindemittel als Pulver zum Einrieseln, als Folie oder als Gittemetz oder in Form von Bindefasern. Flüssige Bindemittel sind gelöst in Wasser oder organischen Lösemittel oder als Dispersion applizierbar. Überwiegend werden zur adhäsiven Verfestigung Bindedispersionen gewählt: Duroplasten in Form von Phenol- oder Melaminharzdispersionen, Elastomere als Dispersionen natürlicher oder synthetischer Kautschuke oder meist Dispersionen von Thermoplasten wie Acrylate, Vinylacetate, Polyurethane, Styrol-Butadien-Systeme, PVC u. ä. sowie deren Copolymere. Im Normalfall handelt es dabei um anionische oder nicht-ionogen stabilisierte Dispersionen, in besonderen Fällen können aber auch kationische Dispersionen von Vorteil sein.

Die Art des Bindemittelauftrages kann gemäß dem Stand der Technik erfolgen und ist beispielsweise in Standardwerken der Beschichtung oder der Vliestechnik wie "Vliesstoffe" (Georg Thieme Verlag, Stuttgart, 1982) oder "Textiltechnik-Vliesstofferzeugung" (Arbeitgeberkreis Gesamttextil, Eschborn, 1996) nachzulesen.

Für mechanisch vorverfestigte Vliese, die bereits eine ausreichende Verbundfestigkeit aufweisen, bietet sich der einseitige Sprühauftrag eines Bindemittels an, um Oberflächeneigenschaften gezielt zu verändern.

Neben dem sparsamen Umgang mit dem Bindemittel wird bei derartiger Arbeitsweise auch der Energiebedarf zur Trocknung deutlich reduziert. Da keine Abquetschwalzen benötigt werden und die Dispersionen vorwiegend in dem oberen Bereich des Vliesstoffes verbleibt, kann eine unerwünschte Verhärtung und Versteifung des Vlieses weitgehend verhindert werden.

Für eine ausreichende adhäsive Verfestigung des Vliesträgers ist im allgemeinen Bindemittel in der Größenordnung von 1 % bis 50 %, insbesondere 3 % bis 20 %, bezogen auf das Gewicht des Faservlieses, zuzugeben.

Die Zugabe des Bindemittels kann bereits bei der Vliesherstellung, bei der mechanischen Vorverfestigung oder aber in einem gesonderten Prozessschritt erfolgen, wobei dieser inline oder offline durchgeführt werden kann. Nach der Bindemittelzugabe muss temporär für das Bindemittel ein Zustand erzeugt werden, in dem dieses klebend wird und adhäsiv die Fasern verbindet - dies kann während der Trocknung zum Beispiel von Dispersionen, aber auch durch Erwärmung erreicht werden, wobei über flächige oder partielle Druckanwendung weitere Variationsmöglichkeiten gegeben sind. Die Aktivierung des Bindemittels kann in bekannten Trockenkanälen, bei geeigneter Bindemittelauswahl aber auch mittels Infrarotstrahlung, UV-Strahlung, Ultraschall, Hochfrequenzstrahlung oder dergleichen erfolgen. Für die spätere Endanwendung ist es sinnvoll, aber nicht zwingend notwendig, dass das Bindemittel nach Ende des Vlies-Herstellprozesses seine Klebrigkeit verloren hat.

Eine weitere Sonderform der adhäsiven Verfestigung besteht darin, dass die Aktivierung des Bindemittels durch Anlösen oder Anquellen erfolgt. Prinzipiell können hierbei auch die Fasern selbst oder zugemischte Spezialfasern die Funktion des Bindemittels übernehmen. Da für die meisten polymeren Fasern derartige Lösemittel jedoch aus Umweltgesichtspunkten bedenklich beziehungsweise problematisch in ihrer Handhabung sind, wird dieses Verfahren eher selten angewandt.

Als Ausgangsmaterialien für den textilen Träger sind insbesondere Polyester-, Polypropylen-, Viskose- oder Baumwollfasern vorgesehen. Die vorliegende Erfindung ist aber nicht auf die genannten Materialien beschränkt, sondern es können, für den Fachmann erkenntlich ohne erfinderisch tätig werden zu müssen, eine Vielzahl weiterer Fasern zur Herstellung des Vlieses eingesetzt werden.

Weiterhin sind auch Maschenwaren hervorragend geeignet. Maschenwaren sind textile Flächengebilde hergestellt aus einem oder mehreren Fäden oder Fadensystemen durch Maschenbildung (Fadenschleifen), im Unterschied zu Webwaren (Geweben), bei der die Fläche durch Verkreuzen von zwei Fadensystemen (Kett- und Schussfäden) hergestellt wird und den Vliesen (Faserverbundstoffen), bei denen ein loser Faserflor durch Wärme, Nadelung, Nähen oder durch Wasserstrahlen verfestigt wird.

Maschenwaren lassen sich in Gestricke, bei denen die Fäden in Querrichtung durch das Textil laufen, und in Gewirke einteilen, bei denen die Fäden längs durch das Textil laufen. Maschenwaren sind durch ihre Maschenstruktur prinzipiell nachgiebige, anschmiegsame Textilien, weil sich die Maschen in Länge und Breite dehnen können und das

Bestreben haben, in ihre Ausgangslage zurückzukehren. Sie sind bei hochwertigem Material sehr strapazierfähig.

Schließlich kann das bahnförmige Material ein beidseitig antiadhäsiv beschichtetes Material sein wie ein Trennpapier oder eine Trennfolie. Gegebenfalls wird das beschichtete Trägermaterial mittels einer weiteren Trennfolie oder einem weiteren Trennpapier eingedeckt.

Die Dicke der Selbstklebemasse auf dem bahnförmigen Material kann zwischen 10 μm und 2000 μm betragen, vorzugsweise zwischen 20 μm und 500 μm, besonders bevorzugt zwischen 50 und 400 μm.

Beispiele

Anhand des folgenden Beispieles soll die Erfindung näher beschrieben werden, ohne damit die Erfindung einschränken zu wollen.

Zur kontinuierlichen lösungsmittelfreien Herstellung eines Prototypen einer mit Wirkstoff dotierten Selbstklebemasse diente ein Doppelschneckenextruder der Firma Leistritz (Schneckendurchmesser von 50 mm, Unterteilung in 13 Zonen). Innerhalb aller Zonen kann der Extruder beliebig mit Förder-, Misch- und Knetelementen bestückt werden.

Es werden die Rohstoffkomponenten in spezieller Reihenfolge und Position dosiert (s. Abbildung 1). Hierfür kommen unterschiedliche Dosiersysteme zum Einsatz. Die pulverförmigen Stoffe oder Granulate werden über gravimetrische Feststoffdosierer (z. B. der Fa. K-tron oder Fa. Gericke) dem Doppelschneckenextruder direkt oder über Seitenbeschickungen (SB) zugeführt. Die flüssigen Komponenten wie auch der Wirkstoff werden über Zahnradpumpen exakt dosiert. Hierbei wird die Dosierrate über Dosierwaagen kontrolliert und geregelt. Die Zuführung der Harze erfolgt in flüssiger Form als Schmelze. Es kommen Schmelzgeräte mit Zahnradpumpen der Firmen Inatec, HBB und Robatech zum Einsatz.

Beispielrezeptur (alle Angaben in Teilen pro Hundert (phr) bezogen auf die Summe des Naturkautschukanteils):

A) 100,0 phr Naturkautschuk Crepe

B) 22,7 phr hydriertes Kohlenwasserstoffharz (Escorez 5380, ExxonMobil)

C) 55,9 phr hydriertes Kolophoniumharz (Foral AX-E, Eastman)

D) 7,3 phr Mineralöl (Pionier 2071, Hansen&Rosenthal)

E) 2,6 phr Alterungsschutzmittel (Lowinox 22M46, Great Lakes Chemical Corp.)

F) 8,9 phr Weichmacher (Cetiol V, Henkel KGaA)

G) 18,6 phr Capsicumextrakt als hyperämisierender Wirkstoff, entsprechend 0,4 phr Capsaicinoide (berechnet als Capsaicin)

In der Abbildung 1 ist eine schematische Übersicht über die zur Durchführung des Verfahrens verwendete Anlage gezeigt.

Die Rohstoffe A und E wurden jeweils über eine gravimetrische Dosierung dem Füllteil (1) eines Doppelschnekkenextruders zugeführt.

Das Material wurde über eine erste fördernde Verfahrenszone (2) mit einem Teil (5 %) der Rohstoffe B und C vermischt. In den weiteren Zonen (3) - (4) folgen abwechselnd Knet- und Förderzonen.

In die fördernde Verfahrenszone (5) wurden - rezepturabhängig - die Komponenten B+C gravimetrisch zudosiert. Anschließend wurde gemischt und gefördert (6)-(9).

Danach folgte in Zone (10), eine gravimetrische Dosierung der Komponente G.

Über volumetrisch arbeitende Zahnradpumpen erfolgte in Zone (12) die zudosiert der Rohstoffe D und F.

In den Zonen (12) - (13) wurde das Material gemischt und gefördert.

Anschließend wurde die Selbstklebemasse durch eine Zahnradpumpe (14) über eine Breitschlitzdüse (15) ausgeformt und ausgetragen. In einem Glättwerk (16) wurde die Masse auf den Träger (T1) (vgl. Abbildung 3) beschichtet und nachfolgend auf den Träger (T2) kaschiert.

Die Drehzahl des Extruders betrug zwischen 100 und 150 rpm. Am Austritt aus dem Extruder hatte die Masse eine Temperatur zwischen 90 °C und 100 °C.

Die Beschichtung des Trägermaterials erfolgte mit einer Breitschlitzdüse.

Aus den hergestellten Labormustern wurden jeweils 5 Proben mit einem Durchmesser von 2,2 cm ausgestanzt und auf ihr Freisetzungsverhalten auf Schweinehaut untersucht.

Hierzu wurde eine Probe auf ein Stück Schweinehaut appliziert, das auf ein Freisetzungsgefäß nach Franz

gelegt wurde. Das Freisetzungsgefäß war mit einer Rezeptorphase gefüllt, die auf konstante 35,5 °C temperiert war und ständig gerührt wurde. Nach 24 h wurde der Gehalt an Capsaicinoiden in der Haut und der Rezeptorphase quantitativ bestimmt.

Das aus der in Beispiel 1 beschriebenen Masse hergestellte Pflaster zeigte eine gute Freisetzung des Wirkstoffs. Unter den oben genannten Bedingungen wurden 14 Promille des Wirkstoffs dermal absorbiert. Der normierte, relative Anteil der dermal absorbierten Menge teilt sich dabei folgendermaßen auf:

1,6% in der Hornschicht

44,2% in der Epidermis

52,2% in der Dermis

2,0% in der Rezeptorphase

**Patentansprüche**

**1.** Verfahren zur kontinuierlichen und lösungsmittelfreien Herstellung von Selbstklebemassen auf Basis von Naturkautschuk und/oder Naturkautschukderivaten mit mindestens einem pharmazeutischen Wirkstoff in einem kontinuierlich arbeitenden Aggregat mit einem Füll- und einem Compoundierteil, bestehend aus

a) Vorlage von Naturkautschuk und/oder Naturkautschukderivat, gegebenenfalls mit einem Trennhilfsmittel in den Füllteil des Aggregats, gegebenenfalls Vorlage von niedermolekularem Synthesekautschuk, Füllstoffen, Weichmachern, Tackifiern, Harzen und/oder Additiven,
b) Übergabe der Vorlagekomponenten der Selbstklebemasse aus dem Füllteil in den Mastikations- und Compoundierteil des Extruders,
c) Zugabe mindestens eines Stabilisators im Bereich des Mastikations- und Compoundierteils,
d) Zugabe von mindestens einem pharmazeutischen Wirkstoff und gegebenenfalls Zugabe der nicht in dem Füllteil aufgegebenen Komponenten der Selbstklebemasse wie, niedermolekulares SBC, Füllstoffe, Weichmacher, Tackifier, Harze und/oder Additive,
e) Herstellung einer homogenen Selbstklebemasse durch eine Kombination aus Förder-Misch- und Knetelementen im Compoundierteil
f) Austragen der Selbstklebemasse.

**2.** Verfahren nach den Ansprüchen 1 und 2, **dadurch gekennzeichnet, dass** das Aggregat ein Doppelschneckenextruder mit mindestens einer Dosieröffnung, vorzugsweise zwischen zwei und sieben, und mindestens einer Entgasungsöffnung ist.

**3.** Verfahren nach zumindest einem der vorherigen Ansprüche, **dadurch gekennzeichnet, dass** in den Mastikations- und Compoundierteil des Aggregates kein Sauerstoff und/oder sauerstoffhaltiges Gas eingeleitet wird.

**4.** Verfahren nach zumindest einem der vorherigen Ansprüche, **dadurch gekennzeichnet, dass** im Aggregat eine Temperatur von 65 bis 220 °C herrscht, wobei die Temperatur am Anfang des Extruders höher ist als am Ende.

**5.** Verfahren nach zumindest einem der vorherigen Ansprüche, **dadurch gekennzeichnet, dass** zwischen Aggregat und Beschichtungsvorrichtung eine Schmelzepumpe oder ein Extruder zur Förderung der Selbstklebemasse angeordnet ist.

**6.** Verfahren nach zumindest einem der vorherigen Ansprüche, **dadurch gekennzeichnet, dass** der Eintrag von Sauerstoff in den Mastikations- und Compoundierteil vermieden wird, insbesondere durch Schutzgase vermieden wird.

**7.** Verfahren nach zumindest einem der vorherigen Ansprüche, **dadurch gekennzeichnet, dass** Selbstklebemasse nach dem Austragen auf ein bahnförmiges Material, auf eine Trennfolie oder auf ein Trennpapier beschichtet wird und gegebenenfalls mittels einer weiteren Trennfolie oder einem weiteren Trennpapier eingedeckt wird.

**8.** Verfahren nach Anspruch 6, **dadurch gekennzeichnet, dass** die Beschichtung des bahnförmigen Materials mit

einer Extrusionsdüse erfolgt.

**9.** Verfahren nach Anspruch 6, **dadurch gekennzeichnet, dass** die Beschichtung des bahnförmigen Materials mit einem Walz- oder Kalanderwerk erfolgt, wobei die Selbstklebemasse bei Durchgang durch einen oder mehrere Walzenspalte auf die gewünschte Dicke geformt wird.

**10.** Verfahren nach Anspruch 6, **dadurch gekennzeichnet, dass** die Beschichtung des bahnförmigen Materials mit einer Extrusionsdüse und einem Walz- oder Kalanderwerk erfolgt, wobei die Selbstklebemasse bei Durchgang durch einen oder mehrere Walzenspalte auf die gewünschte Dicke geformt wird.

**11.** Verfahren nach zumindest einem der vorherigen Ansprüche, **dadurch gekennzeichnet, dass** die Dicke der Selbstklebemasse auf dem bahnförmigen Material zwischen 10 μm und 2000 μm beträgt, vorzugsweise zwischen 20 μm und 500 μm, besonders bevorzugt zwischen 50 und 400 μm.

**12.** Verfahren nach zumindest einem der vorherigen Ansprüche, **dadurch gekennzeichnet, dass** der pharmazeutische Wirkstoffanteil in der Selbstklebemasse zwischen 0,001 Gew.-% und 10 Gew.-%, bevorzugt zwischen 0,01 Gew.-% und 7 Gew.-%, besonders bevorzugt zwischen 0,03 Gew.-% und 5 Gew.-%.

**13.** Lösungsmittefreie, wirkstoffhaltige Selbstklebemasse, erhältlich nach einen Verfahren der Ansprüche 1 bis 6.

**14.** Medizinisches Trägermaterial, erhältlich nach mindestens einem der Ansprüche 7 bis 12.

Abbildung 1

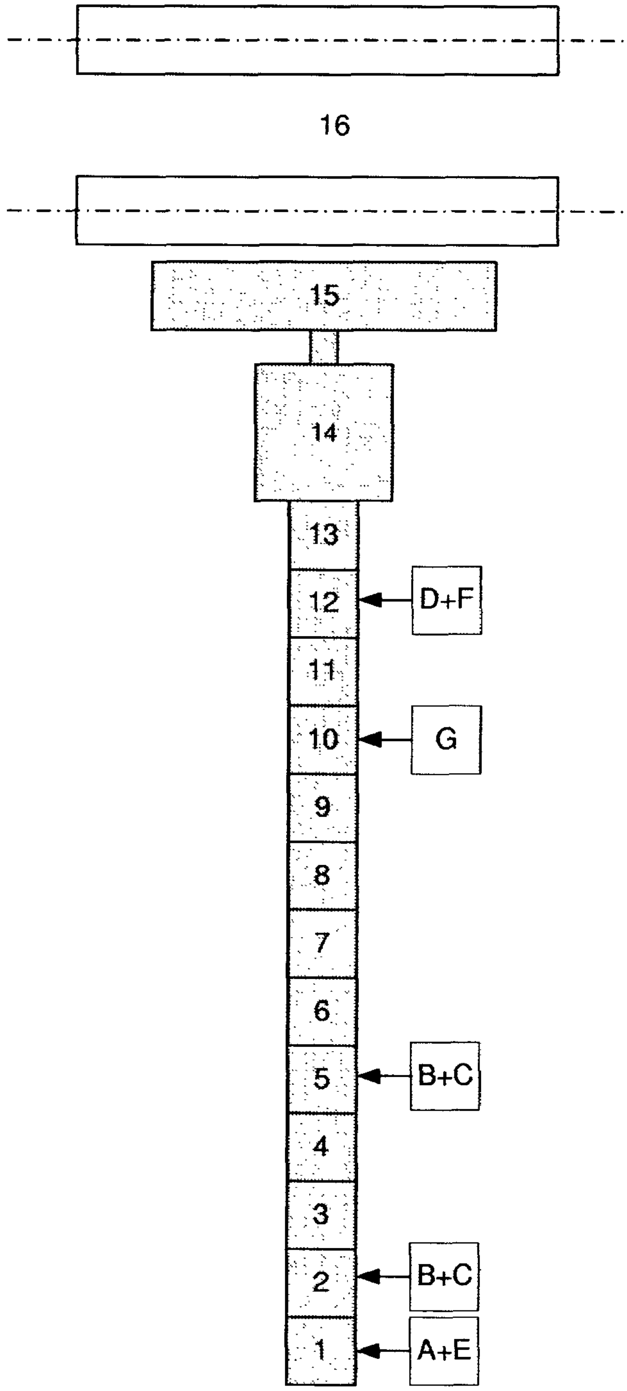

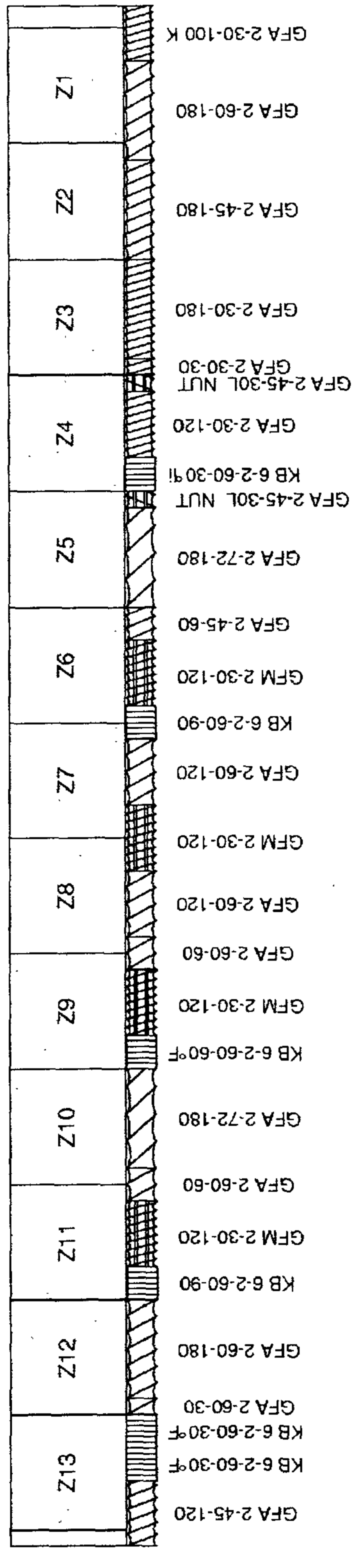
Z1
Z2
Z3
Z4
Z5
Z6
Z7
Z8
Z9
Z10
Z11
Z12
Z13
GFA 2-30-100 K
GFA 2-60-180
GFA 2-45-180
GFA 2-30-180
GFA 2-30-30
GFA 2-45-30L NUT
GFA 2-30-120
KB 6-2-60-30
GFA 2-45-30L NUT
GFA 2-72-180
GFA 2-45-60
GFM 2-30-120
KB 6-2-60-90
GFA 2-60-120
GFM 2-30-120
GFA 2-60-120
GFA 2-60-60
GFM 2-30-120
KB 6-2-60-60°F
GFA 2-72-180
GFA 2-60-60
GFM 2-30-120
KB 6-2-60-90
GFA 2-60-180
GFA 2-60-30
KB 6-2-60-30°F
KB 6-2-60-30°F
GFA 2-45-120

Abbildung 2

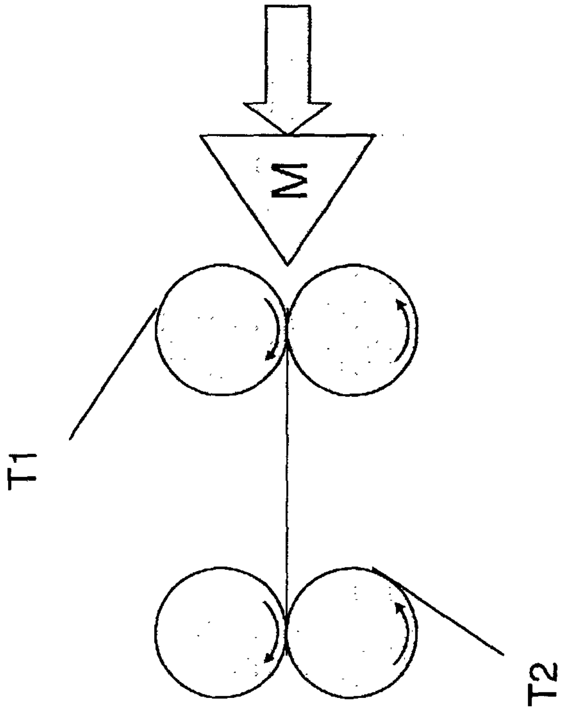


Abbildung 3

Europäisches Patentamt

# EUROPÄISCHER RECHERCHENBERICHT

Nummer der Anmeldung
EP 06 10 0057

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 6 166 110 A (BREDAHL ET AL) 26. Dezember 2000 (2000-12-26) * Spalte 6, Zeile 39 - Zeile 46; Abbildungen 1,2 * * Spalte 10, Zeile 45 - Spalte 11, Zeile 42; Anspruch 16 * * Spalte 13, letzter Absatz * | 1-6,8, 11,13 | INV. B29C47/10 B29C47/40 B32B37/15 C09J7/02 A61K9/70 |
| X | US 6 632 522 B1 (HYDE PATRICK DARBY ET AL) 14. Oktober 2003 (2003-10-14) * Absätze [0037], [0042], [0043], [0073], [0074], [0148], [0149], [0193]; Ansprüche 1,3,39; Abbildung 13 * | 1,7,13, 14 | |
| X | DE 19 54 214 A1 (PYTON AG) 6. Mai 1971 (1971-05-06) * Abbildungen 1,2; Beispiele 1-3 * | 1,13 | |
| D,A | US 5 539 033 A (BREDAHL ET AL) 23. Juli 1996 (1996-07-23) * Anspruch 30; Abbildungen 1-4 * | 1 | RECHERCHIERTE SACHGEBIETE (IPC) |
| A | EP 1 116 763 A (BEIERSDORF AKTIENGESELLSCHAFT) 18. Juli 2001 (2001-07-18) * Ansprüche 1-12 * | 1,2,4,5, 11,12 | B29C C09J A61K B32B C08J |
| A | EP 1 078 968 A (BEIERSDORF AG) 28. Februar 2001 (2001-02-28) * Anspruch 1; Abbildungen 1-3 * | 1,5,7-10 | |
| A | EP 1 186 395 A (CWW-GERKO AKUSTIK GMBH & CO. KG) 13. März 2002 (2002-03-13) * Abbildung 1 * | 8,10 | |
| A | EP 1 077 091 A (TESA AG) 21. Februar 2001 (2001-02-21) * Abbildung 1 * | 7-10 | |

Der vorliegende Recherchenbericht wurde für alle Patentansprüche erstellt

| Recherchenort | Abschlußdatum der Recherche | Prüfer |
|---|---|---|
| Den Haag | 17. Mai 2006 | Van Nieuwenhuize, O |

1

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 06 10 0057

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

17-05-2006

| Im Recherchenbericht angeführtes Patentdokument | | Datum der Veröffentlichung | Mitglied(er) der Patentfamilie | | | Datum der Veröffentlichung |
|---|---|---|---|---|---|---|
| US 6166110 | A | 26-12-2000 | CA | 2257752 | A1 | 24-05-2000 |
| | | | DE | 19856996 | A1 | 25-05-2000 |
| | | | JP | 2000160127 | A | 13-06-2000 |
| US 6632522 | B1 | 14-10-2003 | US | 2002007014 | A1 | 17-01-2002 |
| DE 1954214 | A1 | 06-05-1971 | KEINE | | | |
| US 5539033 | A | 23-07-1996 | AU | 674183 | B2 | 12-12-1996 |
| | | | AU | 5402994 | A | 08-06-1994 |
| | | | BR | 9307396 | A | 24-08-1999 |
| | | | CA | 2147507 | A1 | 26-05-1994 |
| | | | CN | 1087570 | A | 08-06-1994 |
| | | | DE | 69320359 | D1 | 17-09-1998 |
| | | | DE | 69320359 | T2 | 21-01-1999 |
| | | | EP | 0668819 | A1 | 30-08-1995 |
| | | | ES | 2122044 | T3 | 16-12-1998 |
| | | | JP | 8503243 | T | 09-04-1996 |
| | | | MX | 9306773 | A1 | 31-01-1995 |
| | | | WO | 9411175 | A1 | 26-05-1994 |
| EP 1116763 | A | 18-07-2001 | AT | 263225 | T | 15-04-2004 |
| | | | AU | 772442 | B2 | 29-04-2004 |
| | | | AU | 7225200 | A | 19-07-2001 |
| | | | DE | 10001546 | A1 | 19-07-2001 |
| | | | ES | 2215555 | T3 | 16-10-2004 |
| | | | US | 2001039302 | A1 | 08-11-2001 |
| EP 1078968 | A | 28-02-2001 | DE | 19939075 | A1 | 22-02-2001 |
| | | | JP | 2001106999 | A | 17-04-2001 |
| EP 1186395 | A | 13-03-2002 | KEINE | | | |
| EP 1077091 | A | 21-02-2001 | DE | 19939073 | A1 | 22-02-2001 |
| | | | JP | 2001098232 | A | 10-04-2001 |
| | | | US | 6506447 | B1 | 14-01-2003 |

EPO FORM P0461

Für nähere Einzelheiten zu diesem Anhang : siehe Amtsblatt des Europäischen Patentamts, Nr.12/82